**Europäisches Patentamt**

(19) **European Patent Office**

**Office européen des brevets**

(11) Veröffentlichungsnummer: **0 063 744**
**B1**

(12) ## EUROPÄISCHE PATENTSCHRIFT

(45) Veröffentlichungstag der Patentschrift:
**29.01.86**

(51) Int. Cl.⁴: **C 12 M 1/02,** B 01 L 3/00,
B 01 L 7/00, A 01 G 33/00

(21) Anmeldenummer: **82103130.9**

(22) Anmeldetag: **14.04.82**

(54) **Verfahren und Vorrichtung zur Serienkultivierung von Mikroorganismen.**

(30) Priorität: **18.04.81 DE 3115745**

(43) Veröffentlichungstag der Anmeldung:
**03.11.82 Patentblatt 82/44**

(45) Bekanntmachung des Hinweises auf die Patenterteilung:
**29.01.86 Patentblatt 86/5**

(84) Benannte Vertragsstaaten:
**CH FR LI NL**

(56) Entgegenhaltungen:
DE - A - 2 139 608
FR - A - 2 449 891
GB - A - 1 112 919
US - A - 2 622 183
US - A - 2 829 528
US - A - 3 338 794
US - A - 3 649 464
US - A - 4 002 009
US - A - 4 058 370
US - A - 4 061 315
US - A - 4 284 725

PRODUCT INFORMATION, DYNATECH
LABORATORIES LTD, Billinghurst, Sussex, GB, "AM89
Shaker incubator"
PRODUCT INFORMATION, DYNATECH

(73) Patentinhaber: **Kernforschungsanlage Jülich
Geselischaft mit beschränkter Haftung, Postfach 1913,
D-5170 Jülich 1 (DE)**

(72) Erfinder: **Halfenberg, Rolf, Otto-Molz-Strasse 2,
D-7800 Freiburg (DE)**
Erfinder: **Payer, Hans-Dieter, Dr., Unteranger 13 c
(Grossinzemoos), D-8061 Röhrmoos (DE)**
Erfinder: **Soeder, Carl Johannes, Prof. Dr.,
Diemelstrasse 5, D-4600 Dortmund 41 (DE)**

(56) Entgegenhaltungen: (Fortsetzung)
**LABORATORIES LTD.
COOKE MICROTITER SYSTEM, DYNATECH
COMPANIES, GB; "A.M. 69 microshaker"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf ein Verfahren zur sterilen Serienkultivierung von lebenden Zellen oder Mikroorganismen, insbesondere Algen, in flüssigen Nährmedien sowie auf eine Vorrichtung zur Durchführung des Verfahrens.

Die Kultur von lebenden Zellen oder Mikroorganismen und insbesondere von Mikroalgen gewinnt in der theoretischen und angewandten Biologie immer mehr an Bedeutung. Im Bereich des Gewässerschutzes werden Mikroalgen nach Vorkultur unter definierten Bedingungen für sogenannte Biotestverfahren eingesetzt, um z.B. in Wasserproben qualitativ das Vorliegen von toxischen Substanzen (Umweltgiften) nachzuweisen. Bei solchen und anderen Untersuchungen sind grosse Versuchsserien erforderlich.

Ein wesentliches Problem solcher Versuchsserien besteht in der Reproduzierbarkeit der Kulturbedingungen von einem Ansatz zum folgenden, so dass durch ungleichmässige Bedingungen hervorgerufene Schwankungen im Kulturergebnis ausgeschlossen werden können. Wichtig ist dabei vor allem die Vermeidung einer Sedimentation der Algen in der Suspension, so dass Licht-, Temperatur- und Nährstoffgradienten in derselben gering gehalten und die Stoffwechselvorgänge zwischen Medium und Zellen möglichst günstig gestaltet werden. Zu diesem Zweck wird eine dauernde Turbulenz in der Suspension angestrebt.

Bislang werden für die Serienkultivierung von Mikroorganismen in flüssigen Nährmedien sogenannte Röhrenkulturen vorgesehen, bei denen die Sedimentation der Mikroorganismen durch Einleitung von Gas in die einzelnen Röhren verhindert wird. Bei diesem Verfahren wird zum einen keine absolute Gleichmässigkeit der Kulturen untereinander erreicht, und zum anderen sind nur eine begrenzte Anzahl von Kulturansätzen parallel zu verarbeiten (üblicherweise arbeitet man mit 8 Parallelproben).

Bei den ebenfalls bekannten Erlenmeyer-Schüttelkulturen sind die Lichtverhältnisse bei den verschiedenen Kulturen durch Abschattung der Kulturansätze untereinander nicht absolut gleich und es sind Schwankungen in der Verteilung der Mikroorganismen in der Suspension möglich. Ferner sind auch hier nur sehr begrenzte Probenzahlen parallel verarbeitbar.

Ziel der Erfindung ist daher ein Verfahren zur Serienkultivierung von Mikroorganismen, mit dem grössere Mengen von Parallelproben unter möglichst vergleichbaren Bedingungen verarbeitet werden können.

Das zu diesem Zweck entwickelte erfindungsgemässe Verfahren der eingangs genannten Art ist dadurch gekennzeichnet, dass man die Mikroorganismen bzw. Zellen in Mikrotiterplatten kultiviert, die bei einheitlicher Temperatur gehalten und einer Rüttelbewegung ausgesetzt werden, bei der die Platte ständig parallel zu sich selbst bleibt.

Auf diese Weise lassen sich grosse Serien von Parallelansätzen unter exakt gleichen Bedingungen kultivieren.

Wichtig ist, dass sämtliche Ansätze einer Testserie eine einheitliche Turbulenz erfahren, die durch eine an allen Aufnahmeplätzen der Mikrotiterplatten einheitliche Rüttelbewegung hervorgerufen wird, worunter eine Vibrationsbewegung verstanden wird, der die gesamte Platte ausgesetzt ist. Um zu gewährleisten, dass tatsächlich sämtliche Aufnahmeplätze ähnlichen Bedingungen unterworfen werden, muss daher die Mikrotiterplatte während ihrer Bewegung stets zu sich selbst parallel bleiben und keine Schwenk- oder Drehbewegungen ausführen, die unterschiedliche Auslenkungen vom Ruhepunkt an den einzelnen Aufnahmeplätzen mit sich bringen würde.

Vorzugsweise erfolgt die Bewegung der Mikrotiterplatte nur in einer Ebene.

Eine solche Bewegung kann beispielsweise mit einem an sich bekannten Rütteltisch hervorgerufen werden, an dessen zentralem in Schwingungen versetzten Kreuzkopf die zur Tischmitte weisenden Ecken von vier Mikrotiterplatten gehaltert sind, die durch einen starren Rahmen in starrer Verbindung mit dem Kreuzkopf gehalten werden, der an Schwenk- oder Drehbewegungen gehindert ist.

Die von einem Motor hervorgerufene Rüttelbewegung verursacht zwangsläufig eine lokale Erwärmung und führt so zu Temperaturunterschieden in unterschiedlicher Entfernung von der Wärmequelle. Solche Temperaturunterschiede müssen sicher vermieden werden, weshalb eine für einen angemessenen Temperaturausgleich sorgende Wärmeabschirmung und/oder Kühlung oder Temperierung vorgesehen sein muss.

Zweckmässigerweise wird dafür die Auflagefläche für die Mikrotiterplatten mit einer kombinierten Wärmeisolierung und Flüssigkeitskühlung versehen.

Für die Mikrokulturen dienen zum Beispiel käufliche Mikrotiterplatten aus klarem Kunststoff mit 24 zylinderförmigen Vertiefungen von je 3 ml Fassungsvermögen, deren Böden 2 mm höher liegen als der auf der Unterlage aufstehende Rand. Auf diese Weise kommen die durchsichtigen Böden nicht mit der Unterlage in Berührung und können nicht verkratzt werden, so dass eine optische Analyse der Kulturansätze in vertikalem Strahlengang möglich ist, wie weiter unten näher beschrieben wird. Bei gleichzeitiger Verwendung von 4 solchen Mikrotiterplatten können somit beispielsweise 96 Kulturansätze gleichzeitig bewältigt werden.

Um eine Verdunstung und Infektion durch Fremdorganismen zu verhindern, werden die Kulturplatten mit einer Klarsichtklebefolie überzogen, die sowohl für Kohlendioxid als auch für Sauerstoff gut durchlässig ist. Besonders bewährt hat sich dabei Zelluloseacetatfolie.

Um möglichst einheitliche Kulturbedingungen herbeizuführen, werden die Kulturen ferner insbesondere einem einheitlichen Lichteinfall unter Verwendung von flächenhaft verteilten Leuchtkörpern ausgesetzt.

Eine sämtliche Kulturflächen überdeckende Begasungshaube mit Bohrungen dient schliesslich zur einheitlichen Gasbeaufschlagung aller Kulturansätze.

Weitere Besonderheiten der Erfindung gehen aus den Unteransprüchen und der nachfolgenden Beschreibung eines Ausführungsbeispieles hervor. Diese Beschreibung nimmt Bezug auf die angefügten Zeichnungen, und zwar zeigen:

Figur 1 ein Schema für einen Rütteltisch zur Behandlung von 4 Mikrotiterplatten;

Figur 2 die Zusammensetzung der temperierten Unterlage für die Mikrotiterplatten;

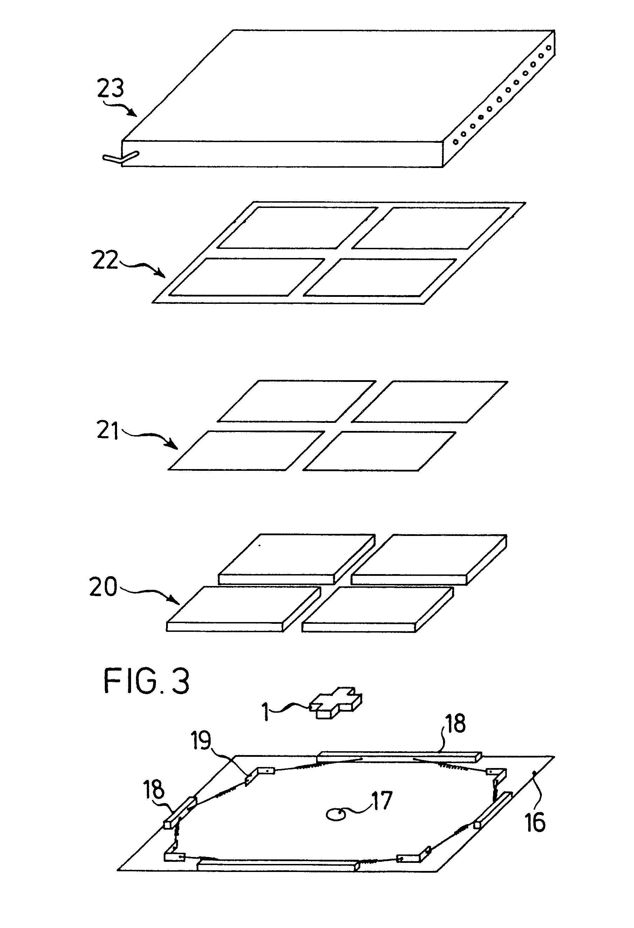

Figur 3 die Rütteltischplatte nebst den auf ihr angeordneten Kulturplatten, Rahmen und Begasungshaube und

Figur 4 einen Ausschnitt aus einem Schreiberdiagramm aus einer Versuchsreihe mit unterschiedlich stark gewachsenen Algenproben.

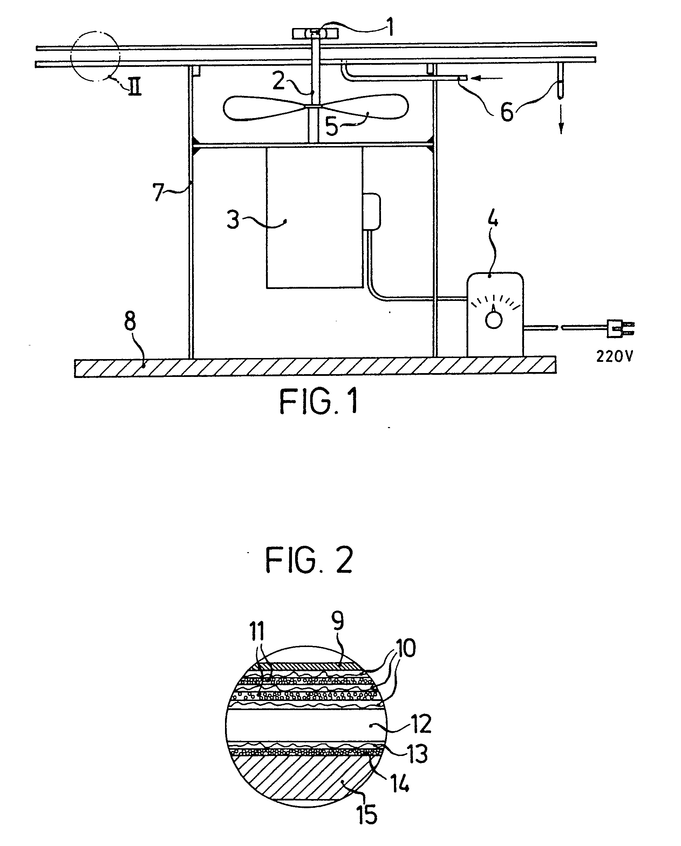

Figur 1 zeigt den zur Aufnahme der Mikrotiterplatten vorgesehenen Rütteltisch mit einem zweifach gelagerten Kreuzkopf 1 mit Excenterwelle 2 der von einem Gleichstrommotor 3 mit stufenlos einstellbarer Drehzahl angetrieben wird. Mit diesem Motor 3 ist ein Gleichrichter und Drehzahlregler 4 verbunden. Oberhalb des Motors sorgt ein Ventilatorflügel 5 für die Verteilung der Motorwärme. Ferner ist die Auflageplatte des Rütteltisches, deren Zusammensetzung mehr im einzelnen in Fig. 2 im Schnitt dargestellt ist, mit einer Flüssigkeitskühlung beziehungsweise -temperierung mit Zu- und Ablauf 6 versehen. Motor, Ventilator und Anschlüsse sind von einem Gehäuse 7 umgeben, das auf der Grundplatte 8 ruht.

Der in Fig. 2 im Schnitt dargestellte Aufbau der Rütteltischplatte umfasst eine mit Teflon beschichtete Edelstahlplatte 9, an die sich abwechselnde Schichten aus Aluminiumfolie 10 und Styropor 11 von etwa 1 mm Dicke anschliessen. Darunter befindet sich eine spiralförmig verlegte Kühlschlange 12, unter der erneut eine Alumi-umfolie 13 und eine Styroporschicht 14 auf der Tragplatte 15 aus PVC vorgesehen sind.

Die vorstehend in ihrem Aufbau näher beschriebene Rütteltischplatte 16 hat eine zentrale Öffnung 17 zur Durchführung der Kreuzkopfwelle sowie neben Halteleisten 18 längs der Kanten mit Federn versehene Einspannecken 19. Auf diesem Rütteltisch werden 4 Kulturplatten 20 eingespannt, deren zur Mitte weisende Ecken an den entsprechenden Winkeln des Kreuzkopfes 1 anliegen. Diese Kulturplatten werden einzeln mit Klarsicht-Klebefolie 21 überzogen, die für Kohlendioxid und Sauerstoff durchlässig ist, jedoch eine Verunreinigung der Kulturansätze verhindert.

Ein starrer Rahmen 22 dient schliesslich zur Fixierung der Kulturplatten, die auf diese Weise gezwungen werden, die horizontale Kreisbewegung des Kreuzkopfes mitzumachen, wobei sie stets zu sich selbst parallel bleiben.

Über den mit Folie abgedeckten Platten wird schliesslich eine Begasungshaube 23 aus Plexiglas vorgesehen, die eine Vielzahl von Bohrungen für die Gasbeaufschlagung der einzelnen Kulturansätze aufweist. Diese Haube ist durchsichtig, so dass eine gleichmässige Belichtung der Kulturansätze durch eine darüber angeordnete Lichtbank mit 8 Leuchtstoffröhren (Osram L20/22R cool white de Luxe) möglich ist. Die Lichtintensität an der Oberfläche der Kulturen beträgt 6000 Lux. Die Positionen der Leuchtstofflampen werden so justiert, dass die Kulturfläche homogen ausgeleuchtet ist.

Um während der Versuche konstante Licht- und Temperaturbedingungen zu gewährleisten, stehen die Kulturplatten nebst Rüttler und Begasungseinrichtung in einem Lichtthermostaten mit Heiz- und Kühlelementen. Zur Vermeidung von Kälte- bzw. Wärmestaus an den Kühl- bzw. Heizelementen wird die Luft über einen Lüfter dauernd umgewälzt.

Die gesamte Anordnung dient insbesondere für Serienuntersuchungen des Einflusses von Schadstoffen und Schadstoffkonzentrationen auf die Entwicklung von Mikroorganismen, insbesondere von Algen. Als Mass für das Algenwachstum wird bei den Versuchen mit Mikrokulturen die Extinktion verwendet. Gemessen wird diese Extinktion im vertikalen Strahlengang bei einer Wellenlänge von 540 nm in den Kultureinheiten auf einem Dünnschicht-Scanner. Bei dem Dünnschicht-Scanner handelt es sich um ein Transportgerät, das auf einem Messtisch die zu messenden Objekte – hier die 24 Kultur-Einheiten auf einer Kulturplatte – zwischen Lichtquelle und Photometermesskopf hindurchführt. Die Bewegung verläuft zweidimensional, mäanderförmig und wird durch ein Programm gesteuert. Die Extinktionswerte werden auf einem Schreiber in Form von Peaks aufgeschrieben.

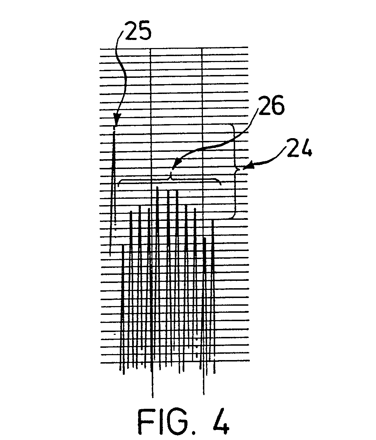

Als Mass für die Konzentration wird die Differenz 24 (in mm) zwischen der Peakhöhe 25 des Wasserwertes (Nullwert) und der 26 der Probe angegeben. Der in Fig. 4 wiedergegebene Ausschnitt zeigt die Peaks 26 von 11 Proben.

Um Fremd- und Streulicht bei der Messung abzuschirmen, wir die Kulturplatte in eine Lochschablone aus Aluminium gestellt.

Linseneffekte an den Rändern der Kultureinheiten werden dadurch vermieden, dass die Kulturplatten mit einer Lochschablone abgedeckt werden.

## Patentansprüche

1. Verfahren zur sterilen Serienkultivierung von lebenden Zellen oder Mikroorganismen, insbesondere von Algen, in flüssigen Nährmedien, dadurch gekennzeichnet, dass man die Mikroorganismen bzw. Zellen in Mikrotiterplatten kultiviert, die bei einheitlicher Temperatur gehalten und einer Rüttelbewegung ausgesetzt werden, bei der die Platte ständig parallel zu sich selbst bleibt.

2. Verfahren nach Anspruch 1, dadurch gekennzeichnet, dass man die Platten auf einer einheitlich temperierten Unterlage rüttelt.

3. Verfahren nach Anspruch 1 oder 2, dadurch gekennzeichnet, dass man die mit gasdurchlässiger Folie abgedeckten Kulturen einheitlich begast.

4. Verfahren nach einem der vorangehenden Ansprüche, dadurch gekennzeichnet, dass die Kulturen einem einheitlichen Lichteinfall ausgesetzt werden.

5. Verfahren nach einem der vorangehenden Ansprüche, dadurch gekennzeichnet, dass transparente Mikrotiterplatten verwendet werden und die Aktivitätsmessung der Zellsuspensionsdichten in den Kultureinheiten in vertikalem Strahlengang erfolgt.

6. Verfahren nach einem der vorangehenden Ansprüche, dadurch gekennzeichnet, dass die Aktivitätsmessung mit Hilfe eines Scanners sowie von zwei die Zwischenräume und Ränder der Kulturaufnahmeplätze der Mikrotiterplatten abdeckenden Lochschablonen erfolgt.

7. Vorrichtung zur Kultivierung von Mikroorganismen oder Zellen nach Anspruch 1, gekennzeichnet durch einen an sich bekannten Rütteltisch mit einer solchen Halterung für zumindest eine Mikrotiterplatte, die einen einheitlichen Bewegungsablauf an allen Punkten der Platte(n) sicherstellt und einer Thermostatisierung der Mikrotiterplatten-Auflagefläche sowie durch eine die Plattenfläche überdeckende Begasungshaube.

8. Vorrichtung nach Anspruch 7, gekennzeichnet durch einen an sich bekannten, im Zentrum des Rütteltischs angeordneten, an Dreh- oder Schwenkbewegungen gehinderten Kreuzkopf zur Aufnahme der zur Tischmitte hinweisenden Ecken von vier Mikrotiterplatten und durch einen starren Rahmen, der die Platten einzeln in starrer Verbindung zum Kreuzkopf hält.

9. Vorrichtung nach Anspruch 7 oder 8, gekennzeichnet durch eine Temperiereinrichtung unter der Rütteltischplatte, die aus einem spiralförmigen Wärmeaustauscher sowie aus geeigneten wärmeleitenden und wärmedämmenden Schichten aufgebaut ist.

**Claims**

1. Process for the sterile series cultivation of living cells or microorganisms, especially of algae, in liquid nutrient media, characterised in that the microorganisms or cells are cultivated in microtitration plates which are kept at uniform temperature and are subjected to a shaking movement wherein the plate continually remains parallel to itself.

2. Process according to claim 1, characterised in that the plates are shaken on a support with uniform temperature control.

3. Process according to claim 1 or 2, characterised in that the cultures covered with gas-pervious foil are uniformly gassed.

4. Process according to one of the preceding claims, characterised in that the cultures are subjected to uniform light incidence.

5. Process according to one of the preceding claims, characterised in that transparent microtitration plates are used, and activity measurement of the cell suspension densities in the culture units is effected with a vertically directed path of rays.

6. Process according to one of the preceding claims, characterised in that activity measurement is effected with the use of a scanner and also two apertured templates which cover the intervening spaces and edges of the culture accommodation areas of the micro-titration plates.

7. Apparatus for the cultivation of microorganisms or cells according to claim 1, characterised by a per se known shaking table with such a holder for at least one micro-titration plate as to ensure uniform movement patterns at all points of the plate(s), and with thermostatting of the micro-titration plate support surface, also by a gassing hood which covers the plate surface.

8. Apparatus according to claim 7, characterised by a per se known crosshead which is situated in the centre of the shaking table and which is prevented from performing rotary or swivelling movements, provided for receiving those corners of four micro-titration plates which are directed towards the middle of the table, and by a rigid frame which holds the plates individually in rigid connection with the crosshead.

9. Apparatus according to claim 7 or 8, characterised by a temperature control device under the shaking table plate, constructed with a spiral heat exchanger and with suitable heat-conducting and heat-insulating layers.

10. Apparatus according to one of claims 7 to 9, characterised by a plexiglass hood for the gassing of cultures, through which a gassing tube provided with holes is taken and which comprises holes for the exhaust gas.

**Revendications**

1. Procédé de culture stérile en série de cellules vivantes ou de microorganismes, notamment d'algues, dans des milieux nutritifs liquides, caractérisé en ce qu'il consiste à cultiver les microorganismes ou les cellules dans des plaques de microtitration qui sont maintenues à une température uniforme et qui sont soumises à un mouvement de vibration suivant lequel la plaque reste constamment parallèle à elle-même.

2. Procédé suivant la revendication 1, caractérisé en ce qu'il consiste à faire vibrer les plaques sur un support à température équilibrée de manière uniforme.

3. Procédé suivant la revendication 1 ou 2, caractérisé en ce qu'il consiste à insuffler de manière uniforme du gaz sur les cultures recouvertes d'une feuille perméable au gaz.

4. Procédé suivant l'une des revendications précédentes, caractérisé en ce qu'il consiste à soumettre les cultures à une lumière incidente uniforme.

5. Procédé suivant l'une des revendications précédentes, caractérisé en ce qu'il consiste à utiliser des plaques de microtitration transparentes et à effectuer la mesure de l'activité des densités de suspension des cellules dans les unités de culture avec un trajet du rayonnement qui est vertical.

6. Procédé suivant l'une des revendications précédentes, caractérisé en ce qu'il consiste à effectuer la mesure de l'activité à l'aide d'un scanographe, ainsi que de deux gabarits à perforations recouvrant les espaces intermédiaires et les bords des emplacements de réception des cultures des plaques de microtitration.

7. Appareil de culture de microorganismes ou de cellules suivant la revendication 1, caractérisé par une table vibrante connue en soi, ayant une fixation pour au moins une plaque de microtitration qui assure un mouvement uniforme en tous points de la plaque ou des plaques, et une thermostatisation de la surface de support des plaques de microtitration, ainsi qu'une hotte d'insufflation de gaz qui recouvre la surface des plaques.

8. Appareil suivant la revendication 7, caractérisé par un croisillon en soi connu, disposé au centre de la table vibrante, empêché d'effectuer des mouvements de rotation ou de basculement et destiné à recevoir les sommets tournés vers le milieu de la table de quatre plaques de microtitration et par un cadre rigide qui maintient individuellement les plaques en liaison rigide par rapport au croisillon.

9. Appareil suivant la revendication 7 ou 8, caractérisé par un dispositif d'équilibrage de la température disposé sous le plateau de la table vibrante, et constitué d'un échangeur de chaleur en forme de spirale, ainsi que de couches convenables conductrices de la chaleur et calorifuges.

10. Appareil suivant l'une des revendications 7 à 9, caractérisé par une hotte en plexiglass pour insuffler des gaz sur les cultures, à travers laquelle passe un tuyau d'insufflation de gaz muni de perçages et qui présente des perçages pour le gaz résiduaire.

# FIG. 1

# FIG. 2

FIG. 3

FIG. 4